(19) Europäisches Patentamt
European Patent Office
Office européen des brevets

(11) Numéro de publication: **0 167 553**
**B1**

(12) **FASCICULE DE BREVET EUROPÉEN**

(45) Date de publication du fascicule du brevet: **21.03.90**

(51) Int. Cl.[5]: **A 61 K 31/12,** C 07 C 49/84

(21) Numéro de dépôt: **85900141.4**

(22) Date de dépôt: **24.12.84**

(86) Numéro de dépôt international: **PCT/FR 84/00302**

(87) Numéro de publication international: **WO 85/02768 (04.07.85 Gazette 85/15)**

# (54) NOUVELLE APPLICATION THERAPEUTIQUE D'UN DERIVE DE L'INDANE DIONE ET LES COMPOSITIONS PHARMACEUTIQUES DESTINEES A CET USAGE.

(30) Priorité: **26.12.83 FR 8320755**

(43) Date de publication de la demande: **15.01.86 Bulletin 86/03**

(45) Mention de la délivrance du brevet: **21.03.90 Bulletin 90/12**

(84) Etats contractants désignés: **AT BE CH DE FR GB LI LU NL SE**

(56) Documents cité: **FR-A-2 382 424**

(73) Titulaire: **Laboratoires BOUCHARD**
**6, rue Anna-Jacquin**
**F-92102 Boulogne - Billancourt (FR)**

(72) Inventeur: **PETIBON, Guy**
**27, rue Gutenberg**
**F-92100 Boulogne - Billancourt (FR)**

(74) Mandataire: **Bourgognon, Jean-Marie**
**Cabinet Flechner 22, Avenue de Friedland**
**F-75008 Paris (FR)**

**EP 0 167 553 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe dípposition (Art. 99(1) Convention sur le brevet européen).

LIBERGRAF, STOCKHOLM 1990

**Description**

L'invention a pour objet l'utilisation thérapeutique d'un dérivé de l'Indane dione et les compositions pharmaceutiques servant à cette utilisation.

L'invention a plus particulièrement pour objet l'utilisation comme agent normolipémiant d'un dérivé de la ninhydrine avec une acétophenone.

L'invention a spécifiquement pour objet l'emploi comme agent normolipémiant du 2-[(3, 4-dimethoxybenzoyl) methyl] 2-hydroxy 1,3-dioxo indane. Ce composé répond à la formule

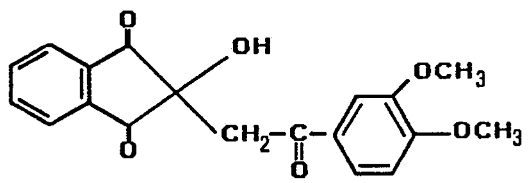

Il a déjà été décrit dans le brevet Français 2.382.424, déposé au nom des mêmes ayant-droit. Il se trouve revendiqué globalement dans le cadre d'une formule générale susceptible de l'inclure.

Le dit brevet Français mentionne également à titre d'application industrielle le fait que les composés de la formule générale ont des propriétés thérapeutiques remarquables et notamment des propriétés anti-inflammatoires, analgésiques et spasmolytiques.

Le dit brevet Français comprend donc les médicaments nouveaux définis comme contenant comme principe actif un ou plusieurs des composés de la formule générale.

La présente invention a pour objet l'utilisation du 2-[(3, 4-dimethoxybenzolyl) methyl] 2-hydroxy-1,3-dioxo indane pour la préparation d'un médicament ayants des propietés normolipémiantes. Cette nouvelle application thérapeutiques est surprenante, car il est peu usuel qu'un produit anti-inflammatoire analgésique ou spasmolytique manifeste de telles propriétés. Elle est, en outre, encore plus surprenante du fait qu'elle se manifeste à des doses où le produit en question ne manifeste pas de propriétés anti-inflammatoires ou analgésiques. C'est ainsi que le 2 [(3, 4-dimethoxybenzoyl) methyl] 2-hydroxy 1,3-dioxo indane a été préalablement testé comme anti-inflammatoire à la dose de 250 mg/kg, pour entraîner un effet liminaire anti-inflammatoire. C'est ainsi que la posologie prévue au brevet Français 2.382.424 pour l'administration humaine devait s'échelonner de 400 à 1.200 mg par jour.

Il a été mis en évidence, plus récemment, le fait que le 2-[(3, 4-dimethyoxybenzoyl) methyl] 2-hydroxy 1, 3-dioxo indane dès la dose de 100 mg/kg manifestait une nette activité anti-lipémiante, et que sur un test aussi sévère que l'hyperlipémie provoquée par le Triton WR 1339®, chez le Rat, celle-ci était antagonisée au diminuée dès la dose de 125 mg/kg par voie orale.

Par ailleurs, le composé en question ne manifestant qu'une très faible toxicité, l'administration peut se faire à des doses plus élevées sans crainte d'effets toxiques ou pendant des périodes très prolongées

L'action normolipémiante du 2-[(3, 4-dimethoxybenzoyl) methyl] 2-hydroxy 1,3-dioxo indane se manifeste aussi bien sur le plan biochimique (taux de cholestérol total, taux de cholesterol estérifié, teneur en lipo-protéines haute densité (HDL), teneur en lipo-protéines basse-densité (LDL) lipides totaux et triglycerides, que sur le plan de la regression histologique.

Les examens macroscopiques effectués sur des animaux rendus atheromateux par un régime riche en cholesterol mettent en évidence, pour les animaux recevant en même temps le produit de l'invention, une moindre fréquence des lésions macroscopiques, donc un moindre pourcentage d'animaux atteints, et une intensité moindre des lésions observées.

Le produit de l'invention a été comparé sur ces tests au Clofibrate, et au Fenclofibrate, administrés aux mêmes doses. Le 2-[(3, 4-dimethoxybenzoyl) methyl] 2-hydroxy 1,3-dioxo indane s'avère être aussi actif que le Clofibrate sur les paramètres biochimiques, et plus actif sur l'évalution des lésions macroscopiques.

L'invention concerne également les compositions pharmaceutiques destinées au traitement de l'hyperlipidémie, de l'hypercholesterolémie et de l'atheromatose caractérisées en ce qu'elles renferment de 50 à 200 mg de principe actif par unité de prise.

D'une manière préférée, les compositions pharmaceutiques selon l'invention renferment de 100 à 200 mg de 2-[(3, 4-dimethoxybenzoyl) methyl] 2-hydroxy 1,3-dioxo indane en association au en mélange avec un excipient, ou véhicule inerte non toxique pharmaceutiquement compatible.

La posologie journalière s'échelonne chez l'adulte de 300 à 600 mg par jour, répartis en 3 à 6 administrations.

Les formes pharmaceutiques selon l'invention sont celles qui conviennent pour l'administration par voie parentérale, orale ou rectale. A cette fin, elles sont présentées sous forme de comprimés nus ou enrobés, de dragées, de capsules, de tablettes secables ou non, de comprimés à enrobages protecteurs, de comprimés à libération progressive, de microcapsules à enrobage entérique, de capsules molles, de gelules, de pilules, de cachets, de solutions ou suspensions buvables, de solutés injectables ou de suppositoires.

Les exemples suivants illustrent l'invention. Ils ne la limitent en aucune façon.

**Exemple 1.**

Etude toxicologique du 2-[(3, 4-dimethoxybenzoyl) methyl] 2-hydroxy 1, 3 - dioxo indane.

**I. METHODOLOGIE**

**1.1. ANIMAUX**

Souris SWISS mâles et femelles de 22 à 25g de poids corporel. Animaux EOPS. (exempte d'organisme pathogène spécifique), en provenance de l'élevage CERJ., 53680 LE GENEST et acclimatés au moins 8 jours à l'animalerie du laboratoire.

**1.2. CONDITIONS D'HEBERGEMENT**

Sauf mention contraire précisée dans les tableaux de résultats, 10 animaux par dose, sexe et cage en MAKROLON SAFI® de 40/25 cm. Litière de copeaux de bois blanc. Alimentation standard pour rats et souris UAR et eau ordinaire à volonté.

**1.3. TRAITEMENTS.**

Ils sont administrés sans jeûne préalable:

- par voie digestive (intubation gastrique par sonde oesophagienne métallique) à raison de 0,5 ml ou 1 ml pour 20 g de poids corporel d'une suspension aqueuse à 3p.100 en gomme arabique du dérivé testé.
- par voie intrapéritonéale à raison de 0,5 ml ou 1 ml pour 20 g d'une suspension du dérivé testé dans le soluté isotonique de chlorure de sodium à 3p.100 en gomme arabique.

**1.4. EXAMEN DES ANIMAUX**

Pendant 6 heures après l'administration unique, puis tous les jours pendant 15 jours.

**1.5. EXPRESSION DES RESULTATS**

S'il y a lieu, les doses létales cinquante (DL50) sont calculées selon les méthodes de:

- Behrens et Karber (B.K.)
- Miller et Tainter (M.T.), cette dernière méthode permettant d'attribuer, pour le seuil de probabilité de P = 0,05 un intervalle de confiance aux DL 50.

En outre, sont déterminées les DLO et DL100.

- DLO = DMT: Dose Maxima toujours tolérée
- DL100 = DmM: Dose minima toujours Mortelle.

**2. RESULTATS**

**2.1. VOIE INTRAPERITONEALE**

| Produit mg/kg IP | Volume Administré ml/20g | Concentration g p. 100 | Létalite sur Mâles | 10 souris Femelles |
|---|---|---|---|---|
| 2000 | 0,5 | 8 | 0 | 0 |
| 2500 | 0,5 | 10 | 1 | 1 |
| 3000 | 0,5 | 12 | 3 | 6 |
| 3500 | 0,5 | 14 | 5 | 8 |
| 4000 | 0,5 | 16 | 8 | 10 |
| 4500 | 0,5 | 18 | 10 | 10 |
| 5000 | 0,5 | 20 | 10 | 10 |

| | Souris Mâles | Souris Femelles |
|---|---|---|
| DLO = | 2000 mg/kg | 2000 mg/kg |
| DL50 = | 3400 mg/kg | 3000 mg/kg (B.K.) |
| | 3350± 190 mg/kg | 3000 ± 145 mg/kg (M.T) |
| DL100 = | 4500 mg/kg | 4000 mg/kg |

*Létalité et symptomatologie*: La létalité n'est jamais immédiate mais survient de 24 à 48 heures après l'injection. Pour les doses moyennes de 3 à 4 g/kg, on a également enregistré quelques morts après 5 à 7 jours, généralement 1 mort

par dose et par sexe.
La symptomatologie initiale repose sur des mouvements de reptation à l'injection puis s'installe une prostation durable avec poil hérissé, bradypnée, cyanose. La mort survient par arrêt respiratoire en 24 - 48 heures. Les souris survivantes demeurent prostrées environ une semaine, du moins pour les doses ayant entraîné une morbidité.

**2.2. VOIE DIGESTIVE**
On a administré une dose unique de produit à des lots de 10 souris mâles et femelles: 10.000 mg/kg à raison de 1 ml pour 20 g de poids corporel.
A. 10.000 mg/kg VO. ni létalité, ni symptomatologie toxique.
DLO ≥ 10.000 mg/kg
DL50 et DL100 > 10000 mg/kg

**3. CONCLUSIONS**
L'étude de la toxicité par administration unique (dite toxicité aiguë) du composé a été menée chez la souris mâle et femelle par voies digestive et intrapéritonéale, comparativement au CLOFIBRATE®.
Par voie digestive, le dérivé n'est pas toxique à la dose de 10000 mg/kg, alors que le CLOFIBRATE® conduit aux DL50 suivantes:
2500 ± 235 mg/kg souris mâles
2650 ± 190 mg/kg souris femelles.
Par voie intrapéritonéale, le dérivé est très sensiblement moins toxique que le CLOFIBRATE®, puisque les DL50 enregistrées sont de l'ordre de:
3000 mg/kg pour le composé de l'invention,
contre 1850 mg/kg (mâles) et 1700 mg/kg (femelles avec le CLOFIBRATE.
En conclusion, le composé de l'invention est moins toxique que le CLOFIBRATE® lors d'administration uniques tant par voie orale que digestive.

**Exemple II**

Etude de l'action hypolipémiante du 2-[(3,4 -dimethoxybenzoyl methyl] 2-hydroxy 1, 3 - dioxo indane.

**I. PRINCIPE DE L'ESSAI**
L'hyperlipidémie par injection de Triton WR 1339 (marque de fabrique), réalisée chez le Rat Wistar, est antagonisée ou diminuée par l'administration d'agents hypolipémiants.

**2. METHODOLOGIE**

a) **Caractères généraux des essais:**

- Rats Wistar mâles.
- Poids corporel: 150 - 180 grammes.
- Animaux E.O.P.S. (Exempts d'Organisme Pathogène Spécifique).
- Alimentation UAR AO4
- Eau de boisson à volonté
- 6 animaux par lot de traitement et par cage MAKROLON SAFI (marque de fabrique) 60 X 40 cm, sur litière de copeaux de bois dépoussiérés
- Cages placées dans un module animalerie climatisé (19 à 21°C) à degré hygrométrique constant (40 à 60 p.100), dans lequel l'air est renouvelé 10 fois par heure, 12 heures d'éclairement par jour.

b) **Déroulement des essais:**

. - 18 h: mise à jeun.
. Temps 0 - administration du Triton WR 1339® (400 mg/kg IP.).
. + 10 mn: administration du produit étudié.
– + 6 h: 1er prélèvement sanguin au sinus rétro-orbitaire.
. + 24 h: 2ème prélèvement sanguin au sinus rétro-orbitaire.

c) **Lots de traitement:**
L'essai comporte 8 lots dont un lot témoin (animaux sains) et un lot témoin triton (Tableau I).

**3. RESULTATS**
Les résultats individuels des examens sanguins pratiqués aux temps 6 heures et 24 heures sont les résultats des pourcentages par rapport au Procétofène à 250 mg/kg et sont regroupés dans les tableaux II et III.

**TABLEAU II**

Produit de l'invention - Scores par rapport au Procétofène 250 mg/kg - 6 heures.

| Produit | Doses mg/kg | Chol. Total | Lipides Totaux | Triglycérides | Chol. HDL/LDL | Score Total |
|---|---|---|---|---|---|---|
| Produit de l'invention | 125 | 2.44 | - | 0.31 | - | 2.75 |
| - dito - | 250 | 1.15 | 0.92 | 0.80 | 0.87 | 3.74 |
| - dito - | 500 | 0.35 | 1.00 | 1.60 | 2.00 | 4.95 |
| - dito - | 750 | 0.78 | 0.14 | 0.80 | - | 1.72 |
| Procétofène | 250 | 1.00 | 1.00 | 1.00 | 1.00 | 4.00 |
| Procétofène | 500 | 1.65 | 1.65 | 1.29 | 1.53 | 5.12 |

**TABLEAU III**

Produit de l'invention - Scores par rapport au Procétofène 250 mg/kg - 24 heures.

| Produit | Doses mg/kg | Chol. Total | Lipides Total | Triglycérides | Chol. HDL/LDL | Score Total |
|---|---|---|---|---|---|---|
| Produit de l'invention | 125 | 0.28 | - | 0.26 | 1.40 | 1.94 |
| - dito - | 250 | 1.13 | 1.56 | 0.85 | 1.10 | 4.64 |
| - dito - | 500 | 0.75 | 1.62 | 0.49 | 0.80 | 3.66 |
| - dito - | 750 | 0.61 | - | 0.72 | 0.40 | 1.73 |
| Procétofène | 250 | 1.00 | 1.00 | 1.00 | 1.00 | 4.00 |
| Procétofène | 500 | 1.17 | 0.85 | 1.01 | 0.30 | 3.33 |

**4. CONCLUSIONS**
L'activité hypolipémiante du composé de l'invention a été étudiée vis-à-vis de l'hyperlipidémie au TRITON WR®, chez le Rat Wistar mâle, en comparaison avec celle du Procétofène.

Aux doses étudiées (250 et 500 mg/kg), l'activité des deux médicaments est proportionnelle à la dose, 6 heures après l'intoxication par le Triton et inversement proportionnelle 24 heures plus tard.

A posologie égale, les 2 produits présentent une similitude d'activité hypolipémiante vis-à-vis de la technique au TRITON®.

**Exemple III**

Activité hypolipémiante et anti-athéromateuse du 2-(3, 4-dimethoxybenzoyl) methyl 2 - hydroxy 1,3 dioxo indane.

**1. METHODOLOGIE**
La technique utilisée est celle, légèrement modifiée, de AUBERT D., FERAUD J.C., LACAZE B., PEPINO., PANAK E. et PODESTA M. "Athérogénèse expérimentale chez le Rat Wistar". ATHEROSCLEROSIS 20 (1974) 263 - 280.

**2. CARACTERES GENERAUX DES ESSAIS**

a) **Animaux - Régime**

- Rats: Wistar femelles, E.O.P.S. en provenance de l'élevage EVIC-CEBA.
- Poids corporel: 160 - 180 g au départ de l'étude
- Alimentation: UAR AO3, enrichi en cholestérol (0,5 %) et en Acide chloique (0,5 %).
- Eau de boisson: eau de ville.

- 15 animaux par lot et par cage MAKROLON SAFI® de 59 x 38,5 cm sur litière de copeaux de bois blanc dépoussiérés.
- Cages placées dans un module animalerie climatisé (20°C ± 1°C), à l'humidité constante (50 % ± 10 %). Durée d'éclairement: 12 heures par jour (7 h à 19 h).
- Administration des produits: Après 15 jours de régime athéromateux, on leur administre par voie orale pendant 4 jours 250 000 Ul/kg de vitamine $D_2$ (Stérogyl 15 H) en solution dans l'huile d'olive. On administre ensuite les produits à étudier pendant 4 semaines dès la fin de l'administration de l'agent lésionnel.

b) **Lots de traitement**
L'étude porte sur 5 Lots de 15 rats (tableau N° IV)

**TABLEAU IV**

| Lots | Traitement |
|---|---|
| 1 | TEMOINS SAINS |
| 2 | TEMOINS RÉGIME |
| 3 | FENCLOFIBRATE® (100 mg/kg |
| 4 | CLOFIBRATE® (100 mg/kg |
| 5 | Composé de l'invention (100 mg/kg) |

c) **Observations**

- *Le comportement* et l'invention pondérale des animaux sont étudiés par l'observation quotidienne et les pesées hebdomadaires. L'étude de l'évolution pondérale ne montre pas de différence significative entre les différents lots. C'est un indice de bonne tolérance du traitement.
- *La moralité* est notée dans chaque lot; elle peut atteindre 30 % des animaux ce qui nécessite des lots de 15 animaux. L'administration chronique de 100 mg/kg de Clofibrate® correspond à la dose maximale tolérée par le Rat sain selon nos expériences personnelles. Dans nos conditions expérimentales le Clofibrate® (4/15) et le Fenclofibrate (3/15) élèvent le taux de mortalité tandis que le produit de l'invention ne s'élève pas par rapport aux Témoins Régime (2/15)

**3. HISTOPATHOLOGIE**
Les aortes, les foies et les coeurs sont fixés au Bouitaqueux et inclus à la paraffine après passage par les alcools à concentration croissante et par le toluène.

Les coeurs sont conservés en blocs de paraffine pour examen ultérieur si nécessaire.

Les foies sont colorés par la technique à l'hématéine-éosine. Les aortes sont colorées par:

- la technique à l'orcéine - vert lumière afin de mettre en évidence les altérations structurales des lames élastiques.
- la technique à l'acide périodique plus réactif de Schiff pour déceler les dépôts de mucopolysaccharides,
- la technique à l'hématéine plus éosine afin de mettre en évidence les dépôts de calcium.

L'intensité des altérations observées et le degré d'athéromatose sont indiquées par l'un des signes:

| | COTATION |
|---|---|
| – Pas d'athéromatose | 0 |
| ± Très léger degré d'athéromatose | 0,5 |
| + Léger degré d'athéromatose | 1 |
| ++ Moyen degré d'athéromatose | 2 |
| +++Important degré d'athéromatose | 3 |

Les zones athéromateuses sont dénombrées sur une longueur identique pour chacune des aortes examinées. Il convient de noter que, sous les termes de zones athéromateuses, on englobe des lésions minimes (rupture d'au moins trois lames élastiques dont les extrêmités sont séparées par un espace envahi par une substance Mac Manus positive ou éventuellement calcifié), jusqu'aux lésions les plus étendues.

**4. EXAMENS BIOCHIMIQUES.**
En fin d'étude, on pratique sur tous les animaux survivants les examens suivants:

- Cholestérol total,
- Cholestérol estérifié,

- Cholestérol HDL
- Cholestérol LDL (évaluation),
- Lipides totaux,
- Triglycérides.

5. **AUTOPSIE**:
En fin d'étude, tous les animaux sont sacrifiés par ponction subtotale du sang à l'aorte abdominale après anesthésie au pentobarbital sodique. On prélève le coeur, le foie et l'aorte sur laquelle on effectue la cotation du pouvoir athérogène. Pour ce faire, les aortes sont coupées longitudinalement puis étalées pour permettre une vue d'ensemble du revêtement endothélial depuis les valvules aortiques jusqu'à la bifurcation du tronc coeliaque (segment inférieur de l'aorte thoracique).
*L'aorte* est séparée en 2 zones:
- Zone 1: Valvules aortiques - premières artères intercostales
- Zone 2: premières artères intercostales - bifurcation du tronc coeliaque.

L'intensité des dépôts athéromateux est évaluée selon les critères suivants:

- Stade 0: Absence de dépôts
- Stade 1: Dépôts discrets (> 1 mm) peu nombreux
- Stade 2: Dépôts discrets nombreux
- Stade 3: Dépôts importants (> 1 mm) nombreux
- Stade 4: Dépôts très importants ou très nombreux mais laissant quelques zones non infiltrées.
- Stade 5: Dépôts sur tout l'endothélium.

6. **RESULTATS**
Les résultats des observations permettent de classer comme suit l'action des produits.
Produit de l'invention > Fenofibrate > Clofibrate

*Examens biochimiques*
Les résultats des examens biochimiques pratiqués sur les animaux sont regroupés dans les tableaux.

Le Tableau V montre les pourcentages de variation par rapport aux témoins régime et donne le score obtenu par produit pour chaque paramètre étudié par rapport au Clofibrate® (Base 1).

Le score général par produit, obtenu en additionnant tous les scores individuels permet la classification suivante Clofibrate (6) > Produit de l'invention (5.6) > Fenclofibrate (1.98)

La protection contre le risque athérogène peut être estimé par les rapports (Tableau VI):

$$\frac{\textbf{CHOLESTEROL T}}{\textbf{CHOLESTEROL HDL}} \quad \text{et} \quad \frac{\textbf{CHOLESTEROL LDL}}{\textbf{CHOLESTEROL HDL}}$$

La classification individuelle est alors la suivante: Clofibrate® (2) > Produit de l'invention (0.90) > Fenclofibrate (0.23)

*Examens macroscopiques* (Tableau VII)
Deux paramètres sont à prendre en considération:

1. Le nombre d'animaux présentant des lésions macroscopiques, dont le pourcentage d'animaux atteints.
2. L'intensité des lésions observées cotées selon les indications fournies précédemment.

La classification individuelle s'inscrit comme suit: Produit de l'invention (2.57) > Fenclofibrate® (2.17) > Clofibrate® (2).

*Examens histopathologiques*
Les examens histologiques ont donné des résultats individuels qui ont été analysés puis regroupés par lot.

Trois paramètres ont été cotés séparément:

1. Le nombre d'animaux présentant des lésions histologiques, donc le pourcentage d'animaux atteints. Ce pourcentage peut être différent de celui observé macroscopiquement, en effet les coupes histologiques peuvent éviter ou inclure certaines zones visibles macroscopiquement. Certaines zones décelables à l'histologie peuvent d'autre part, ne pas l'être lors de l'examen macroscopique.
2. L'intensité des lésions athéromateuses observées, cotées selon les indications fournies précédemment.
3. Le nombre des lésions athéromateuses observées, rapportées à un lot de 15 animaux.

Les résultats de nos observations permettent de classer comme suit les différents produits: Fenclofibrate (2.13) > Clofibrate® (2.00) > Produit de l'invention (1.3)

**TABLEAU N° II**

-Examens Biochimiques

Pourcentage de variation par rapport aux témoins régime et score

| Lot | Cholesterol T % | Score | Cholesterol E % | Score | Cholesterol HDL % | Score | Cholesterol LDL % | Score | Lipides totaux % | Score | Triglycerides % | Score |
|---|---|---|---|---|---|---|---|---|---|---|---|---|
| Tem-Tem | | | | | | | | | | | | |
| Tem Régime | | | | | | | | | | | | |
| Fenclofibrate® | − 1.09 | 0.02 | − 22.08 | 0.40 | + 4.17 | 0.04 | − 8.69 | 0.16 | − 10.31 | 0.29 | − 25.86 | 1.07 |
| Clofibrate® | − 48.75 | 1.00 | − 55.00 | 1.00 | + 104.17 | 1.00 | − 55.37 | 1.00 | − 35.43 | 1.00 | − 24.14 | 1.00 |
| Produit de L'invention | − 55.47 | 1.14 | − 57.70 | 1.05 | − 33.33 | − | − 57.41 | 1.04 | − 48.47 | 1.37 | − 25.86 | 1.07 |

**TABLEAU N° III**

Risque Athérogène

| Lot | Cholesterol T/ Score | % /Tem Régime | Cholesterol HDL Score/Clofibrate | Cholesterol LDL Score | % /Tem Régime | Cholesterol HDL Score/Clofibrate | Score Total |
|---|---|---|---|---|---|---|---|
| Tem-Tem | 7.28 | | | 5.07 | | | |
| Tem Régime | 26.67 | | | 24.46 | | | |
| Fenclofibrate® | 25.32 | − 5.06 | 0.07 | 21.44 | − 12.35 | 0.16 | 0.23 |
| Clofibrate® | 6.69 | − 74.91 | 1.00 | 5.35 | − 78.13 | 1.00 | 2.00 |
| Produit de l'invention | 17.81 | − 33.22 | 0.44 | 15.63 | − 36.10 | 0.46 | 0.90 |

**TABLEAU N° IV**

Observations Macroscopiques

| Lot | Animaux Atteints % | % de diminution Tem Régime | Score Clofibrate® | Intensité des lésions Cotation | % de diminution Tem Régime | Score Clofibrate® | Score Total |
|---|---|---|---|---|---|---|---|
| Tem-Tem | 0 | − | − | − | − | − | − |
| Tem Régime | 100 | − | − | 2.69 | − | − | − |
| Fenclofibrate® | 83.33 | − 16.67 | 0.92 | 1.83 | − 31.97 | 1.25 | 2.17 |
| Clofibrate® | 81.82 | − 18.18 | 1.00 | 2.00 | − 25.65 | 1.00 | 2.00 |
| Produit de l'invention | 69.23 | − 30.77 | 1.69 | 2.08 | − 22.68 | 0.88 | 2.57 |

**7. CONCLUSION**

Après avoir étudié l'activité hypolipémiante et anti-athéromateuse du produit de l'invention vis-à-vis du régime de AUBERT, légèrement modifié, comparativement au Clofibrate® et au Fenclofibrate®, nous pouvons conclure que 100 mg/kg de Clofibrate® correspondent pratiquement à la dose maximale tolérée par le rat, en administration prolongée. A la même dose le produit de l'invention est mieux toléré.

Les scores obtenus par les différents produits étudiés sont les suivants (Tableau V)

**TABLEAU N° V**

| | Fenclofibrate® | Scores Clofibrate® | Produit de l'invention |
|---|---|---|---|
| Biochimie | 1.98 | 6.00 | 5.67 |
| Risque athérogène | 0.23 | 2.00 | 0.90 |
| Macroscopie | 2.17 | 2.00 | 2.57 |
| Histologie | 2.13 | 3.00 | 1.32 |
| Score Total | 6.51 | 13.00 | 10.46 |

Dans nos conditions expérimentales, le produit de l'invention à 100 mg/kg présente des activités hypolipémiantes et anti-athéromateuses notables, très voisines de celles du Clofibrate® (100 mg/kg) mais supérieures à celles du Fenclofibrate® (100 mg/kg).

**Revendications**

1. Utilisation du 2- [3,4 dimethoxybenzoyl) méthyl] 2-hydroxy-1,3-dioxio indane pour la fabrication d'un médicament ayant des propriétés normolipémiantes.

2. Utilisation suivant la revendication 1 dans laquelle le médicament renferme de 50 à 200 mg de 2[(3, 4 - diméthoxybenzoyl) méthyl] 2-hydroxy 1,3 dioxo-indane en association, ou en mélange avec un véhicule ou un excipient inerte non toxique pharmaceutiquement acceptable.

3. Utilisation suivant la revendication 1 ou 2 dans laquelle l'excipient ou le véhicule est l'un de ceux convenant pour l'administration par voie parentérale, orale ou rectale.

4. Utilisation suivant l'une des revendications précédentes dans laquelle la teneur en principe actif varie de 100 à 200 mg par prise unitaire.

**Patentansprüche**

1. Verwendung von 2- [(3,4-Dimethoxybenzoyl)-methyl] -2-hydroxy-1,3-dioxo-indan für die Herstellung eines Medikamentes mit Lipämienormalisierenden Eigenschaften.

2. Verwendung nach Anspruch 1,
wobei das Medikament 50 bis 200 mg 2- [(3,4-Dimethoxybenzoyl)-methyl]-2-hydroxy-1,3-dioxo-indan in Assoziation oder in Mischung mit einem inerten, nicht toxischen, pharmazeutisch brauchbaren Arzneimittelträger bzw. Träger enthält.

3. Verwendung nach Anspruch 1 oder 2,
wobei der Arzneimittelträger bzw. Träger für eine parenterale, orale oder rektale Verabreichung geeignet ist.

4. Verwendung nach einem der vorgenannten Ansprüche,
wobei der Gehalt des Wirkstoffes 100 und 200 mg pro Einnahmeeinheit beträgt.

**Claims**

1. The use of 2-(3, 4-dimethoxy benzoyl) methyl 2-hydroxy 1, 3-dioxo Indane for producing a drug with normolipaemiant properties.

2. The use as claimed in claim 1 wherein the drug includes from 50 to 200 mg of 2-(3, 4-dimethoxy benzoyl) methyl 2-hydroxy 1,3-dioxo Indane in admixture or in conjection with an inert, non toxic pharmaceutically-acceptable vehicule or carrier.

3. The use as claimed in claim 1 or 2 wherein the vehicule or carier is one of those suitable for administration by parenteral, oral or rectal way.

4. The use as claimed in one of the preceeding claims wherein the content of active ingredient ranges from 100 to 200 mg per unit dosage.